# EUROPEAN PATENT APPLICATION

(11) **EP 2 014 776 A2**
(43) Date of publication of application: **14.01.2009**
(21) Application number: 08012765.7
(22) Date of filing: 06.04.2001
(51) Int. Cl.: C12Q 1/68

(54) **Diagnosis of diseases associated with DNA transcription**

(30) Priority: 06.04.2000 DE 10019058; 07.04.2000 DE 10019173; 30.06.2000 DE 10032529; 01.09.2000 DE 10043826
(62) Divisional of application: 01969303.5
(71) Applicant: Epigenomics AG, 10178 Berlin (DE)
(72) Inventor: Olek, Alexander, 12207 Berlin (DE); Piepenbrock, Christian, 10115 Berlin (DE); Berlin, Kurt, 14532 Stahnsdorf (DE)
(74) Representative: Bittner, Thomas L.

(57) **Abstract**

The present invention relates to the chemically modified genomic sequences of genes associated with DNA transcription, to oligonucleotides and/or PNA-oligomers for detecting the cytosine methylation state of genes associated with DNA transcription which are directed against the sequence, as well as to a method for ascertaining genetic and/or epigenetic parameters of genes associated with DNA transcription.

## Description

The levels of observation that have been well studied by the methodological developments of recent years in molecular biology, are the genes themselves, the translation of these genes into RNA, and the resulting proteins. The question of which gene is switched on at which point in the course in of the development of an individual, and how the activation and inhibition of specific genes specific cells and tissues are controlled is correlatable to the degree and character of the methylation of the genes or of the genome. In this respect, pathogenic conditions may manifest themselves in a changed methylation pattern of individual genes or of the genome.

The present invention relates to nucleic acids, oligonucleotides, PNA-oligomers and to a method for the diagnosis and/or therapy of diseases which have a connection with the genetic and/or epigenetic parameters of genes associated with DNA transcription and, in particular, with the methylation status thereof.

### Prior Art

DNA transcription, the process of using genomic DNA as a template for the synthesis of RNA is a complex process. It requires the interaction of various factors, enzymes and sequences. The initiation of DNA transcription requires a complex known as the basal transcription apparatus consisting of RNA polymerase combined with various factors. They form a complex at the startpoint from which transcription proceeds. Examples of such factors include TFIID, TRF1 and TRF2. This is a major control point for gene expression.

Transcription factors are often classified according to their DNA binding domain. Members of the same group have sequence variations of a specific motif that confer specificity for individual target sites. Common categories include steroid receptors, zinc finger motifs, helix-tum-helix motifs, helix-loop-helix motifs and leucine zippers.

The transcription activity is further supplemented by the binding of other components, such as upstream and inducible factors.

DNA sequence components of the system include both promoter and enhancer elements. Promoter sequences are located in the vicinity of the transcribed region and enhancer elements are located at a distance from the startpoint. These sequences interact with the factors mentioned above to regulate the transcription of RNA.

Further control may be exerted by the action of insulator elements, specialised chromatin structures that have hypersensitive sites. They are able to block passage of any activating or inactivating effects from enhancers, silencers, or LCRs.

A further requirement of DNA transcription is that the DNA be accessible. In most cases the initiation of transcription requires that the DNA be free of nucleosomes. Therefore, a transcription factor, or some other nonhistone protein concerned with the particular function of the site, modifies the properties of a short region of DNA so that nucleosomes are excluded. Several chromatin modification mechanisms are utilised, including histone acetylation and deacetylation activity.

A further parameter that regulates genomic transcription is methylation. Methylation of CpG islands in the regulatory regions of genes has been shown to be a common method of transcription regulation. Furthermore, aberrant methylation patterns have been associated with a variety of disease phenotypes. For example:
- Head and neck cancer: Sanchez-Cespedes M et al. 'Gene promoter hypermethylation in tumours and serum of head and neck cancer patients' Cancer Res. 2000 Feb. 15;60 (4):892-5;
- Hodgkin's disease: Garcia JF et al 'Loss of p 16 protein expression associated with methylation of the p16INK4A gene is a frequent finding in Hodgkin's disease' Lab invest 1999 Dec; 79 (12):1453-9;
- Gastric cancer: Yanagisawa Y et al.' Methylation of the hMLH1 promoter in familial gastric cancer with microsatellite instability' Int J Cancer 2000 Jan 1; 85 (1):50-3;
- Prader-Willi/Angelman's syndrome: Zeschnigh et al 'Imprinted segments in the human genome: different DNA methylation patterns in the Prader Willi/Angelman syndrome region as determined by the genomic sequencing method' Human Mol. Genetics (1997) (6) 3 pp 387-395;
- ICF syndrome: Tuck-Muller et al 'CMDNA hypomethylation and unusual chromosome instability in cell lines from ICF syndrome patients' Cytogenet Call Genet 2000; 89(1-2):121-8;
- Dermatofibroma: Chen TC et al 'Dermatofibroma is a clonal proliferative disease' J Cutan Pathol 2000 Jan;27 (1):36-9);
- Hypertension: Lee SD et al. 'Monoclonal endothelial cell proliferation is present in primary but not secondary pulmonary hypertension' J clin Invest 1998 Mar 1, 101 (5):927-34;
- Autism: Klauck SM et al. 'Molecular genetic analysis of the FMR-1 gene in a large collection of autistic patients' Human Genet 1997 Aug; 100 (2) : 224-9;
- Fragile X Syndrome: Hornstra IK et al. 'High resolution methylation analysis of the FMR1 gene trinucleotide repeat region in fragile X syndrome' Hum Mol Genet 1993 Oct, 2(10):1659-65;
- Huntigton's disease: Ferluga J et al. 'possible organ and age related epigenetic factors in Huntington's disease and colorectal carcinoma' Med hypotheses 1989 May;29(1);51-4;

The proper functioning of the DNA transcription system is essential for the maintenance of cellular functions. Disruptions to the ordered transcription of DNA may impact on a wide variety of disease phenotypes. Mutations in sequences coding for and comprising essential components of the DNA transcription system e.g. zinc finger proteins and enhancer elements have been implicated in a variety of disorders, including cancer:
- Adenosine deaminase deficiency: Ariga T 'Gene therapy for adenosine deaminase (ADA) deficiency: review of the past, the present and the future' Nippon Rinsho 2001 Jan;59(1):72-5;
- Cancer: Garte S, Sogawa K 'Ah receptor gene polymorphisms and human cancer susceptibility' IARC Sci Publ 1999;(148):149-57;
- Hodgkin's disease: Sandvej K, Andresen BS, Zhou XG, Gregersen N, Hamilton-Dutoit S 'Analysis of the Epstein-Barr virus (EBV) latent membrane protein 1 (LMP-1) gene and promoter in Hodgkin's disease isolates: selection against EBV variants with mutations in the LMP-1 promoter ATF-1/CREB-1 binding site' Mol Pathol 2000 Oct;53(5):280-8;
- Ewing's sarcoma family tumors: Aryee DN, Sommergruber W, Muehlbacher K, Dockhorn-Dworniczak B, Zoubek A, Kovar H 'Variability in gene expression patterns of Ewing tumor cell lines differing in EWS-FLI1 fusion type' Lab Invest 2000 Dec;80(12):1833-44;
- Colon cancer: Ishiguro T, Nagawa H, Naito M, Tsuruo T Jpn J ' Inhibitory effect of ATF3 antisense oligonucleotide on ectopic growth of HT29 human colon cancer cells' Cancer Res 2000 Aug;91(8):833-6;
- Breast cancer: Irminger-Finger I, Siegel BD, Leung WC' The functions of breast cancer susceptibility gene 1 (BRCA1) product and its associated proteins' Biol Chem 1999 Feb;380(2):117-28;
- Endometrial cancer: Hori M, Takechi K, Arai Y, Yomo H, Itabashi M, Shimazaki J, Inagawa S, Hori M 'Comparison of macroscopic appearance and estrogen receptor-alpha regulators after gene alteration in human endometrial cancer' Int J Gynecol Cancer 2000 Nov;10(6):469-476;
- Wilms' tumor: Inoue K, Sugiyama H, Ogawa H, Nakagawa M, Yamagami T, Miwa H, Kita K, Hiraoka A, Masaoka T, Nasu K, et al 'WT1 as a new prognostic factor and a new marker for the detection of minimal residual disease in acute leukemia' Blood 1994 Nov 1;84(9):3071-9;
- Solid tumors: Kok K, Naylor SL, Buys CH 'Deletions of the short arm of chromosome 3 in solid tumors and the search for suppressor genes' Adv Cancer Res 1997;71:27-92;
- Leukemia; Tanaka T, Kurokawa M, Ueki K, Tanaka K, Imai Y, Mitani K, Okazaki K, Sagata N, Yazaki Y, Shibata Y, Kadowaki T, Hirai H 'The extracellular signal-regulated kinase pathway phosphorylates AML1, an acute myeloid leukemia gene product, and potentially regulates its transactivation ability'Mol Cell Biol 1996 Jul;16(7):3967-79;
- Polyglutamine disorders: Nucifora FC Jr, Sasaki M, Peters MF, Huang H, Cooper JK, Yamada M, Takahashi H, Tsuji S, Troncoso J, Dawson VL, Dawson TM, Ross CA 'Interference by huntingtin and atrophin-1 with cbp-mediated transcription leading to cellular toxicity' Science 2001 Mar 23;291(5512):2423-8;
- Rheumatoid arthritis: Pascual M, Nieto A, Lopez-Nevot MA, Ramal L, Mataran L, Caballero A, Alonso A, Martin J, Zanelli E 'Rheumatoid arthritis in southern Spain: toward elucidation of a unifying role of the HLA class II region in disease predisposition' Arthritis Rheum 2001 Feb;44(2):307-14;
- Neurodegenerative disorders: Mattila KM, Axelman K, Rinne JO, Blomberg M, Lehtimaki T, Laippala P, Roytta M, Viitanen M, Wahlund L, Winblad B, Lannfelt L'Interaction between estrogen receptor 1 and the epsilon4 allele of apolipoprotein E increases the risk of familial Alzheimer's disease in women' Neurosci Lett 2000 Mar 17;282(1-2):45-8;
- Myelodysplastic syndrome and acute leukemia: Patmasiriwat P, Fraizer G, Kantarjian H, Saunders GF 'WT1 and GATA1 expression in myelodysplastic syndrome and acute leukemia' Leukemia 1999 Jun;13(6):891-900;
- HDR syndrome: Van Esch H, Groenen P, Nesbit MA, Schuffenhauer S, Lichtner P, Vanderlinden G, Harding B, Beetz R, Bilous RW, Holdaway I, Shaw NJ, Fryns JP, Van de Ven W, Thakker RV, Devriendt K 'GATA3 haplo-insufficiency causes human HDR syndrome' Nature 2000 Jul 27;406(6794):419-22;
- Congenital heart disease: Pehlivan T, Pober BR, Brueckner M, Garrett S, Slaugh R, Van Rheeden R, Wilson DB, Watson MS, Hing AV'GATA4 haploinsufficiency in patients with interstitial deletion of chromosome region 8p23.1 and congenital heart disease' Am J Med Genet 1999 Mar 19;83(3):201-6;
- Angiogenesis and erythropoiesis: Krieg M, Haas R, Brauch H, Acker T, Flamme I, Plate KH ' Up-regulation of hypoxia-inducible factors HIF-1 alpha and HIF-2alpha under normoxic conditions in renal carcinoma cells by von Hippel-Lindau tumor suppressor gene loss of function Oncogene 2000 Nov 16;19(48):5435-43;
- Hypertension: Morse JH, Barst RJ, Fotino M, Zhang Y, Flaster E, Fritzler MJ 'Primary pulmonary hypertension: immunogenetic response to high-mobility group(HMG) proteins and histone 'Clin Exp Immunol 1996 Nov;106(2):389-95;
- Myocardial infarction: Hegele RA, Huang LS, Herbert PN, Blum CB, Buring JE, Hennekens CH, Breslow JL 'Apolipoprotein B-gene DNA polymorphisms associated with myocardial infarction.' N Engl J Med 1986 Dec 11;315(24):1509-15;
- Early onset pauciarticular chronic arthritis: Epplen C, Rumpf H, Albert E, Haas P, Truckenbrodt H, Epplen JT 'Immunoprinting excludes many potential susceptibility genes as predisposing to early onset pauciarticular juvenile chronic arthritis except HLA class II and TNF' Eur J Immunogenet 1995 Aug;22(4):311-22;
- AIDS-related non-Hodgkin lymphomas and Hodgkin lymphomas arising in HIV+ patients: Carbone A, Gloghini A, Larocca LM, Capello D, Pierconti F, Canzonieri V, Tirelli U, Dalla-Favera R,Gaidano G ' Expression profile of MUM1/IRF4, BCL-6, and CD138/syndecan-1 defines novel histogenetic subsets of human immunodeficiency virus-related lymphomas' Blood 2001 Feb 1;97(3):744-51;
- Multiple endocrine neoplasia: Calender A 'Molecular genetics of neuroendocrine tumors' Digestion 2000;62 Suppl 1:3-18;
- Myelodysplastic syndrome and acute myeloid leukemia: Xie J, Briggs JA, Morris SW, Olson MO, Kinney MC, Briggs RC 'MNDA binds NPM/B23 and the NPM-MLF1 chimera generated by the t(3;5) associated with myelodysplastic syndrome and acute myeloid leukemia' Exp Hematol 1997 Oct;25(11):1111-7;
- Niemann-Pick type C2 disease: Naureckiene S, Sleat DE, Lackland H, Fensom A, Vanier MT, Wattiaux R, Jadot M, Lobel P' Identification of HE1 as the second gene of Niemann-Pick C disease' Science 2000 Dec 22;290(5500):2298-301;
- Waardenburg syndrome: Bondurand N, Pingault V, Goerich DE, Lemort N, Sock E, Caignec CL, Wegner M, Goossens M 'Interaction among SOX10, PAX3 and MITF, three genes altered in Waardenburg syndrome' Hum Mol Genet 2000 Aug 12;9(13):1907-17;
- Neurological disorders: Geisbrecht BV, Collins CS, Reuber BE, Gould SJ' Disruption of a PEX1-PEX6 interaction is the most common cause of the neurologic disorders Zellweger syndrome, neonatal adrenoleukodystrophy, and infantile Refsum disease' Proc Natl Acad Sci U S A 1998 Jul 21;95(15):8630-5;
- Rieger syndrome: Hjalt T, Amendt B, Murray J 'PITX2 Regulates Procollagen Lysyl Hydroxylase (PLOD) Gene Expression. Implications for the pathology of rieger syndrome' J Cell Biol 2001 Feb 5;152(3):545-52;
- Psoriasis vulgaris: Oka A, Tamiya G, Tomizawa M, Ota M, Katsuyama Y, Makino S, Shiina T, Yoshitome M, Iizuka M, Sasao Y, Iwashita K, Kawakubo Y, Sugai J, Ozawa A, Ohkido M, Kimura M, Bahram S, Inoko H 'Association analysis using refined microsatellite markers localizes a susceptibility locus for psoriasis vulgaris within a 111 kb segment telomeric to the HLA-C gene'Hum Mol Genet 1999 Nov;8(12):2165-70;
- Developmental disorders: Dattani MT, Robinson IC 'The molecular basis for developmental disorders of the pituitary gland in man' Clin Genet 2000 May;57(5):337-46;
- Tuberculosis: Selvaraj P, Narayanan PR, Reetha AM 'Association of vitamin D receptor genotypes with the susceptibility to pulmonary tuberculosis in female patients & resistance in female contacts' Indian J Med Res 2000 May; 111: 172-9;
- Werner syndrome: Kitao S, Lindor NM, Shiratori M, Furuichi Y, Shimamoto A 'Rothmund-thomson syndrome responsible gene, RECQL4: genomic structure and products' Genomics 1999 Nov 1;61(3):268-76;
- Immunological disorders: Puglatti et. al. 'The genes for MHC class II regulatory factors RFX1 and RFX2 are located on the short arm of chromosome 19' Genomics 1992 Aug;13(4):1307-10;
- Hematological disorders: Elbein SC, Teng K, Eddings K, Hargrove D, Scroggin E 'Molecular scanning analysis of hepatocyte nuclear factor 1 alpha (TCF1) gene in typical familial type 2 diabetes in African Americans' Metabolism 2000 Feb;49(2):280-4;
- Sezary syndrome: Showe LC, Fox FE, Williams D, Au K, Niu Z, Rook AH ' Depressed IL-12-mediated signal transduction in T cells from patients with Sezary syndrome is associated with the absence of IL-12 receptor beta 2 mRNA and highly reduced levels of STAT4' J Immunol 1999 Oct 1;163(7):4073-9;
- Viral infection: Yoshida M ' HTLV-1 oncoprotein Tax deregulates transcription of cellular genes through multiple mechanisms' J Cancer Res Clin Oncol 1995;121(9-10):521-8;

The large number of components involved in DNA transcription provide novel targets for therapies and diagnosis for diseases. In particular this may be relevant to diseases where current therapies may have unwanted side effects or fail to provide effective treatment. For cancer patients such methods constitute a considerable advantage over conventional methods such as chemotherapy, which with their massive side effects, sometimes result in unacceptable morbidity or lead up to the death of the patient. In practice, the unwanted side effects associated with cancer therapies frequently limit the treatment which could help a patient.

A global analysis of the status of DNA transcription mechanisms would provide a basis for the development of appropriate and specific therapies for diseases associated with DNA replication. The current state of the art is such that the analysis may be carried out in a gene specific manner based on the results of gene expression, e.g. DNA micro array analysis of mRNA expression or proteomic analysis. The next step would then be to look at the causal factors involved at earlier stages in the regulatory mechanisms controlling DNA transcription. DNA methylation provides a novel level of information at which to analyse the genome.

5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing since 5-methylcytosine has the same base pairing behavior as cytosine. Moreover, the epigenetic information carried by 5-methylcytosine is completely lost during PCR amplification.

A relatively new and currently the most frequently used method for analyzing DNA for 5-methylcytosine is based upon the specific reaction of bisulfite with cytosine which, upon subsequent alkaline hydrolysis, is converted to uracil which corresponds to thymidine in its base pairing behavior. However, 5-methylcytosine remains unmodified under these conditions. Consequently, the original DNA is converted in such a manner that methylcytosine, which originally could not be distinguished from cytosine by its hybridization behavior, can now be detected as the only remaining cytosine using "normal" molecular biological techniques, for example, by amplification and hybridization or sequencing. All of these techniques are based on base pairing which can now be fully exploited. In terms of sensitivity, the prior art is defined by a method which encloses the DNA to be analyzed in an agarose matrix, thus preventing the diffusion and renaturation of the DNA (bisulfite only reacts with single-stranded DNA), and which replaces all precipitation and purification steps with fast dialysis (Olek A, Oswald J, Walter J. A modified and improved method for bisulphite based cytosine methylation analysis. Nucleic Acids Res. 1996 Dec 15;24(24):5064-6). Using this method, it is possible to analyze individual cells, which illustrates the potential of the method. However, currently only individual regions of a length of up to approximately 3000 base pairs are analyzed, a global analysis of cells for thousands of possible methylation events is not possible. However, this method cannot reliably analyze very small fragments from small sample quantities either. These are lost through the matrix in spite of the diffusion protection.

An overview of the further known methods of detecting 5-methylcytosine may be gathered from the following review article: Rein, T., DePamphilis, M. L., Zorbas, H., Nucleic Acids Res. 1998, 26, 2255.

To date, barring few exceptions (e.g., Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) the bisulfite technique is only used in research. Always, however, short, specific fragments of a known gene are amplified subsequent to a bisulfite treatment and either completely sequenced (Olek A, Walter J. The pre-implantation ontogeny of the H19 methylation imprint. Nat Genet. 1997 Nov;17(3):275-6) or individual cytosine positions are detected by a primer extension reaction (Gonzalgo ML, Jones PA. Rapid quantitation of methylation differences at specific sites using methylation-sensitive single nucleotide primer extension (Ms-SNuPE). Nucleic Acids Res. 1997 Jun 15;25(12):252.9-31, WO 95/00669) or by enzymatic digestion (Xiong Z, Laird PW. COBRA: a sensitive and quantitative DNA methylation assay. Nucleic Acids Res. 1997 Jun 15;25(12):2532-4). In addition, detection by hybridization has also been described (Olek et al., WO 99/28498).

Further publications dealing with the use of the bisulfite technique for methylation detection in individual genes are: Grigg G, Clark S. Sequencing 5-methylcytosine residues in genomic DNA. Bioessays. 1994 Jun;16(6):431-6, 431; Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nucleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97/46705, WO 95/15373 and WO 97/45560.

An overview of the Prior Art in oligomer array manufacturing can be gathered from a special edition of Nature Genetics (Nature Genetics Supplement, Volume 21, January 1999), published in January 1999, and from the literature cited therein.

Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example via a confocal microscope. Cy3 and Cy5 dyes, besides many others, are commercially available.

Matrix Assisted Laser Desorption Ionization Mass Spectrometry (MALDI-TOF) is a very efficient development for the analysis of biomolecules (Karas M, Hillenkamp F. Laser desorption ionization of proteins with molecular masses exceeding 10,000 daltons. Anal Chem. 1988 Oct 15;60(20):2299-301). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones.

MALDI-TOF spectrometry is excellently suited to the analysis of peptides and proteins. The analysis of nucleic acids is somewhat more difficult (Gut I G, Beck S. DNA and Matrix Assisted Laser Desorption Ionization Mass Spectrometry. Current Innovations and Future Trends. 1995, 1; 350-57). The sensitivity to nucleic acids is approximately 100 times worse than to peptides and decreases disproportionally with increasing fragment size. For nucleic acids having a multiply negatively charged backbone, the ionization process via the matrix is considerably less efficient. In MALDI-TOF spectrometry, the selection of the matrix plays an eminently important role. For the desorption of peptides, several very efficient matrixes have been found which produce a very fine crystallization. There are now several responsive matrixes for DNA, however, the difference in sensitivity has not been reduced. The difference in sensitivity can be reduced by chemically modifying the DNA in such a manner that it becomes more similar to a peptide. Phosphorothioate nucleic acids in which the usual phosphates of the backbone are substituted with thiophosphates can be converted into a charge-neutral DNA using simple alkylation chemistry (Gut IG, Beck S. A procedure for selective DNA alkylation and detection by mass spectrometry. Nucleic Acids Res. 1995 Apr 25;23(8):1367-73). The coupling of a charge tag to this modified DNA results in an increase in sensitivity to the same level as that found for peptides. A further advantage of charge tagging is the increased stability of the analysis against impurities which make the detection of unmodified substrates considerably more difficult.

Genomic DNA is obtained from DNA of cell, tissue or other test samples using standard methods. This standard methodology is found in references such as Fritsch and Maniatis eds., Molecular Cloning: A Laboratory Manual, 1989.

### Description

The object of the present invention is to provide the chemically modified DNA of genes associated with DNA transcription, as well as oligonucleotides and/or PNA-oligomers for detecting cytosine methylations, as well as a method which is particularly suitable for the diagnosis and/or therapy of genetic and epigenetic parameters of genes associated with DNA transcription. The present invention is based on the discovery that genetic and epigenetic parameters and, in particular, the cytosine methylation pattern of genes associated with DNA transcription are particularly suitable for the diagnosis and/or therapy of diseases associated with DNA transcription.

This objective is achieved according to the present invention using a nucleic acid containing a sequence of at least 18 bases in length of the chemically pretreated DNA of genes associated with DNA transcription according to one of Seq. ID No.1 through Seq. ID No.346 and sequences complementary thereto and/or a chemically pretreated DNA of genes according to the sequences of genes according to table 1 and sequences complementary thereto. In the table, after the listed gene designations, the respective data bank numbers (accession numbers) are specified which define the appertaining gene sequences as unique. GenBank was used as the underlying data bank, which is located at the National Institute of Health at internet address http://www.ncbi.nlm.nih.gov.

The chemically modified nucleic acid could heretofore not be connected with the ascertainment of genetic and epigenetic parameters.

The object of the present invention is further achieved by an oligonucleotide or oligomer for detecting the cytosine methylation state in chemically pretreated DNA, containing at least one base sequence having a length of at least 13 nucleotides which hybridizes to a chemically pretreated DNA of genes associated with DNA transcription according to Seq. ID No.1 through Seq. ID No.346 and sequences complementary thereto and/or a chemically pretreated DNA of genes according to the sequences of genes according to table 1 and sequences complementary thereto. The oligomer probes according to the present invention constitute important and effective tools which, for the first time, make it possible to ascertain the genetic and epigenetic parameters of genes associated with DNA transcription. The base sequence of the oligomers preferably contain at least one CpG dinucleotide. The probes may also exist in the form of a PNA (peptide nucleic acid) which has particularly preferred pairing properties. Particularly preferred are oligonucleotides according to the present invention in which the cytosine of the CpG dinucleotide is the 5^{th} - 9^{th} nucleotide from the 5'-end of the 13-mer; in the case of PNA-oligomers, it is preferred for the cytosine of the CpG dinucleotide to be the 4^{th} - 6^{th} nucleotide from the 5'-end of the 9-mer.

The oligomers according to the present invention are normally used in so called "sets" which contain at least one oligomer for each of the CpG dinucleotides of the sequences of Seq. ID No.1 through Seq. ID No.346 and sequences complementary thereto and/or a chemically pretreated DNA of genes according to the sequences of genes according to table 1 and sequences complementary thereto. Preferred is a set which contains at least one oligomer for each of the CpG dinucleotides from one of Seq. ID No.1 through Seq. ID No.346 and sequences complementary thereto and/or a chemically pretreated DNA of genes according to the sequences of genes according to table 1 and sequences complementary thereto.

Moreover, the present invention makes available a set of at least two oligonucleotides which can be used as so-called "primer oligonucleotides" for amplifying DNA sequences of one of Seq. ID No.1 through Seq. ID No.346 and sequences complementary thereto and/or a chemically pretreated DNA of genes according to the sequences of genes according to table 1 and sequences complementary thereto, or segments thereof.

In the case of the sets of oligonucleotides according to the present invention, it is preferred that at least one oligonucleotide is bound to a solid phase.

The present invention moreover relates to a set of at least 10 n (oligonucleotides and/or PNA-oligomers) used for detecting the cytosine methylation state in chemically pretreated genomic DNA (Seq. ID No.1 through Seq. ID No.346 and sequences complementary thereto and/or a chemically pretreated DNA of genes according to the sequences of genes according to table 1 and sequences complementary thereto). These probes enable diagnosis and/or therapy of genetic and epigenetic parameters of genes associated with DNA transcription. The set of oligomers may also be used for detecting single nucleotide polymorphisms (SNPs) in the chemically pretreated DNA of genes associated with DNA transcription according to one of Seq. ID No.1 through Seq. ID No.346 and sequences complementary thereto and/or a chemically pretreated DNA of genes according to the sequences of genes according to table 1 and sequences complementary thereto.

According to the present invention, it is preferred that an arrangement of different oligonucleotides and/or PNA-oligomers (a so-called "array") made available by the present invention is present in a manner that it is likewise bound to a solid phase. This array of different oligonucleotide- and/or PNA-oligomer sequences can be characterized in that it is arranged on the solid phase in the form of a rectangular or hexagonal lattice. The solid phase surface is preferably composed of silicon, glass, polystyrene, aluminium, steel, iron, copper, nickel, silver, or gold. However, nitrocellulose as well as plastics, such as nylon, which can exist in the form of pellets or also as resin matrices, are possible as well.

Therefore, a further subject matter of the present invention is a method for manufacturing an array fixed to a carrier material for analysis in connection with diseases associated with DNA transcription in which method at least one oligomer according to the present invention is coupled to a solid phase. Methods for manufacturing such arrays are known, for example, from US Patent 5,744,305 by means of solid-phase chemistry and photolabile protecting groups.

A further subject matter of the present invention relates to a DNA chip for the analysis of diseases associated with DNA transcription which contains at least one nucleic acid according to the present invention. DNA chips are known, for example, for US Patent 5,837,832.

Moreover, a subject matter of the present invention is a kit which may be composed, for example, of a bisulfite-containing reagent, a set of primer oligonucleotides containing at least two oligonucleotides whose sequences in each case correspond or are complementary to an 18 base long segment of the base sequences specified in the appendix (Seq. ID No.1 through Seq. ID No.346 and sequences complementary thereto and/or a chemically pretreated DNA of genes according to the sequences of genes according to table 1 and sequences complementary thereto), oligonucleotides and/or PNA-oligomers as well as instructions for carrying out and evaluating the described method. However, a kit along the lines of the present invention can also contain only part of the aforementioned components.

The present invention also makes available a method for ascertaining genetic and/or epigenetic parameters of genes associated with the cycle cell by analyzing cytosine methylations and single nucleotide polymorphisms, including the following steps:

In the first step of the method, a genomic DNA sample is chemically treated in such a manner that cytosine bases which are unmethylated at the 5'-position are converted to uracil, thymine, or another base which is dissimilar to cytosine in terms of hybridization behavior. This will be understood as 'chemical pretreatment' hereinafter.

The genomic DNA to be analyzed is preferably obtained form usual sources of DNA such as cells or cell components, for example, cell lines, biopsies, blood, sputum, stool, urine, cerebral-spinal fluid, tissue embedded in paraffin such as tissue from eyes, intestine, kidney, brain, heart, prostate, lung, breast or liver, histologic object slides, or combinations thereof.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

Fragments of the chemically pretreated DNA are amplified, using sets of primer oligonucleotides according to the present invention, and a, preferably heat-stable polymerase. Because of statistical and practical considerations, preferably more than ten different fragments having a length of 100 - 2000 base pairs are amplified. The amplification of several DNA segments can be carried out simultaneously in one and the same reaction vessel. Usually, the amplification is carried out by means of a polymerase chain reaction (PCR).

In a preferred embodiment of the method, the set of primer oligonucleotides includes at least two olignonucleotides whose sequences are each reverse complementary or identical to an at least 18 base-pair long segment of the base sequences specified in the appendix (Seq. ID No.1 through Seq. ID No.346 and sequences complementary thereto and/or a chemically pretreated DNA of genes according to the sequences of genes according to table 1 and sequences complementary thereto). The primer oligonucleotides are preferably characterized in that they do not contain any CpG dinucleotides.

According to the present invention, it is preferred that at least one primer oligonucleotide is bonded to a solid phase during amplification. The different oligonucleotide and/or PNA-oligomer sequences can be arranged on a plane solid phase in the form of a rectangular or hexagonal lattice, the solid phase surface preferably being composed of silicon, glass, polystyrene, aluminum, steel, iron, copper, nickel, silver, or gold, it being possible for other materials such as nitrocellulose or plastics to be used as well.

The fragments obtained by means of the amplification can carry a directly or indirectly detectable label. Preferred are labels in the form of fluorescence labels, radionuclides, or detachable molecule fragments having a typical mass which can be detected in a mass spectrometer, it being preferred that the fragments that are produced have a single positive or negative net charge for better detectability in the mass spectrometer. The detection may be carried out and visualized by means of matrix assisted laser desorption/ionization mass spectrometry (MALDI) or using electron spray mass spectrometry (ESI).

The amplificates obtained in the second step of the method are subsequently hybridized to an array or a set of oligonucleotides and/or PNA probes. In this context, the hybridization takes place in the manner described in the following. The set of probes used during the hybridization is preferably composed of at least 10 oligonucleotides or PNA-oligomers. In the process, the amplificates serve as probes which hybridize to oligonucleotides previously bonded to a solid phase. The non-hybridized fragments are subsequently removed. Said oligonucleotides contain at least one base sequence having a length of 13 nucleotides which is reverse complementary or identical to a segment of the base sequences specified in the appendix, the segment containing at least one CpG dinucleotide. The cytosine of the CpG dinucleotide is the 5^{th} to 9^{th} nucleotide from the 5'-end of the 13-mer. One oligonucleotide exists for each CpG dinucleotide. Said PNA-oligomers contain at least one base sequence having a length of 9 nucleotides which is reverse complementary or identical to a segment of the base sequences specified in the appendix, the segment containing at least one CpG dinucleotide. The cytosine of the CpG dinucleotide is the 4^{th} to 6^{th} nucleotide seen from the 5'-end of the 9-mer. One oligonucleotide exists for each CpG dinucleotide.

In the fourth step of the method, the non-hybridized amplificates are removed.

In the final step of the method, the hybridized amplificates are detected. In this context, it is preferred that labels attached to the amplificates are identifiable at each position of the solid phase at which an oligonucleotide sequence is located.

According to the present invention, it is preferred that the labels of the amplificates are fluorescence labels, radionuclides, or detachable molecule fragments having a typical mass which can be detected in a mass spectrometer. The mass spectrometer is preferred for the detection of the amplificates, fragments of the amplificates or of probes which are complementary to the amplificates, it being possible for the detection to be carried out and visualized by means of matrix assisted laser desorption/ionization mass spectrometry (MALDI) or using electron spray mass spectrometry (ESI).

The produced fragments may have a single positive or negative net charge for better detectability in the mass spectrometer. The aforementioned method is preferably used for ascertaining genetic and/or epigenetic parameters of genes associated with DNA transcription.

The oligomers according to the present invention or arrays thereof as well as a kit according to the present invention are intended to be used for the diagnosis and/or therapy of diseases associated with DNA transcription by analyzing methylation patterns of genes associated with DNA transcription. According to the present invention, the method is preferably used for the diagnosis and/or therapy of important genetic and/or epigenetic parameters within genes associated with DNA transcription.

The method according to the present invention is used, for example, for the diagnosis and/or therapy of diseases.

The nucleic acids according to the present invention of Seq. ID No.1 through Seq. ID No.346 and sequences complementary thereto and/or a chemically pretreated DNA of genes according to the sequences of genes according to table 1 and sequences complementary thereto can be used for the diagnosis and/or therapy of genetic and/or epigenetic parameters of genes associated with DNA transcription.

The present invention moreover relates to a method for manufacturing a diagnostic agent and/or therapeutic agent for the diagnosis and/or therapy of diseases associated with DNA transcription by analyzing methylation patterns of genes associated with DNA transcription, the diagnostic agent and/or therapeutic agent being characterized in that at least one nucleic acid according to the present invention is used for manufacturing it, possibly together with suitable additives and auxiliary agents.

A further subject matter of the present invention relates to a diagnostic agent and/or therapeutic agent for diseases associated with DNA transcription by analyzing methylation patterns of genes associated with DNA transcription, the diagnostic agent and/or therapeutic agent containing at least one nucleic acid according to the present invention, possibly together with suitable additives and auxiliary agents.

The present invention moreover relates to the diagnosis and/or prognosis of events which are disadvantageous to patients or individuals in which important genetic and/or epigenetic parameters within genes associated with DNA transcription said parameters obtained by means of the present invention may be compared to another set of genetic and/or epigenetic parameters, the differences serving as the basis for a diagnosis and/or prognosis of events which are disadvantageous to patients or individuals.

In the context of the present invention the term "hybridization" is to be understood as a bond of an oligonucleotide to a completely complementary sequence along the lines of the Watson-Crick base pairings in the sample DNA, forming a duplex structure. To be understood by "stringent hybridization conditions" are those conditions in which a hybridization is carried out at 60°C in 2.5 x SSC buffer, followed by several washing steps at 37°C in a low buffer concentration, and remains stable.

The term "functional variants" denotes all DNA sequences which are complementary to a DNA sequence, and which hybridize to the reference sequence under stringent conditions and have an activity similar to the corresponding polypeptide according to the present invention.

In the context of the present invention, "genetic parameters" are mutations and polymorphisms of genes associated with DNA transcription and sequences further required for their regulation. To be designated as mutations are, in particular, insertions, deletions, point mutations, inversions and polymorphisms and, particularly preferred, SNPs (single nucleotide polymorphisms).

In the context of the present invention, "epigenetic parameters" are, in particular, cytosine methylations and further chemical modifications of DNA bases of genes associated with DNA transcription and sequences further required for their regulation. Further epigenetic parameters include, for example, the acetylation of histones which, however, cannot be directly analyzed using the described method but which, in turn, correlates with the DNA methylation.

In the following, the present invention will be explained in greater detail on the basis of the sequences and examples with respect to the accompanying figure without being limited thereto.

### Figure 1

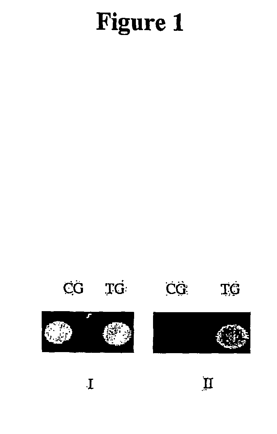

Figure 1 shows the hybridisation of fluorescent labeled amplificates to a surface bound olignonucleotide. Sample I being from a pilocytic astrocytoma tumor sample and sample II being form an oligodenrogliome grade II tumor sample. Flourescence at a spot shows hybridisation of the amplificate to the olignonucleotide. Hybridisation to a CG olgnonucleotide denotes methylation at the cytosine position being analyzed, hybridisation to a TG olignonucleotide denotes no methylation at the cytosine position being analyzed.

### Seq. ID No. 1 through Seq. ID No. 346

Sequences having odd sequence numbers (e.g., Seq. ID No. 1, 3, 5, ...) exhibit in each case sequences of the chemically pretreated genomic DNAs of different genes associated with DNA transcription. Sequences having even sequence numbers (e.g., Seq. ID No. 2, 4, 6, ...) exhibit in each case the sequences of the chemically pretreated genomic DNAs of genes associated with DNA transcription which are complementary to the preceding sequences (e.g., the complementary sequence to Seq. ID No.1 is Seq. ID No.2, the complementary sequence to Seq. ID No.3 is Seq. ID No.4, etc.)

### Seq. ID No. 347 through Seq. ID No. 350

Seq. ID No. 347 through Seq. ID No. 350 show sequences of oligonucleotides used in Example 1.

The following example relates to a fragment of a gene associated with DNA transcription, in this case, CFOS in which a specific CG-position is analyzed for its methylation status.

### Example 1: Methylation analysis in the gene CFOS associated with DNA transcription.

The following example relates to a fragment of the gene CFOS in which a specific CG-position is to be analyzed for methylation.

In the first step, a genomic sequence is treated using bisulfite (hydrogen sulfite, disulfite) in such a manner that all cytosines which are not methylated at the 5-position of the base are modified in such a manner that a different base is substituted with regard to the base pairing behavior while the cytosines methylated at the 5-position remain unchanged.

If bisulfite solution is used for the reaction, then an addition takes place at the non-methylated cytosine bases. Moreover, a denaturating reagent or solvent as well as a radical interceptor must be present. A subsequent alkaline hydrolysis then gives rise to the conversion of non-methylated cytosine nucleobases to uracil. The chemically converted DNA (sequence ID 345) is then used for the detection of methylated cytosines. In the second method step, the treated DNA sample is diluted with water or an aqueous solution. Preferably, the DNA is subsequently desulfonated (10-30 min, 90-100 °C) at an alkaline pH value. In the third step of the method, the DNA sample is amplified in a polymerase chain reaction, preferably using a heat-resistant DNA polymerase. In the present case, cytosines of the gene CFOS are analyzed. To this end, a defined fragment having a length of 951 bp is amplified with the specific primer oligonucleotides TTTTGAGTTTTAGAATTGTT (Sequence ID No. 347) and AAAAACCCCCTACTCATCTA (Sequence ID No. 348). This amplificate serves as a sample which hybridizes to an oligonucleotide previously bonded to a solid phase, forming a duplex structure, for example AAAACATTCGCACCTAAT (Sequence ID No. 349), the cytosine to be detected being located at position 105. of the amplificate. The detection of the hybridization product is based on Cy3 and Cy5 fluorescently labeled primer oligonucleotides which have been used for the amplification. A hybridization reaction of the amplified DNA with the oligonucleotide takes place only if a methylated cytosine was present at this location in the bisulfite-treated DNA. Thus, the methylation status of the specific cytosine to be analyzed is inferred from the hybridization product.

In order to verify the methylation status of the position, a sample of the amplificate is further hybridized to another oligonucleotide previously bonded to a solid phase. Said oligonucleotide is identical to the oligonucleotide previously used to analyze the methylation status of the sample, with the exception of the position in question. At the position to be analyzed said oligonucleotide comprises a thymine base as opposed to a cytosine base i.e. AAAACATTCACACCTAAT (Sequence ID No. 350). Therefore, the hybridisation reaction only takes place if an unmethylated cytosine was present at the position to be analysed. The procedure was carried out on cell samples from 2 patients, sample I being from a pilocytic astrocytoma tumor sample and sample II being from an oligodenrogliome grade II tumor sample.

From the results (Figure 1) it can be seen that Sample I contained both methylated and unmethylated cells and sample II was unmethylated.

### Example 2: Diagnosis of diseases associated with DNA transcription

In order to relate the methylation patterns to one of the diseases associated with DNA transcription, it is initially required to analyze the DNA methylation patterns of a group of diseased and of a group of healthy patients. These analyses are carried out, for example, analogously to Example 1. The results obtained in this manner are stored in a database and the CpG dinucleotides which are methylated differently between the two groups are identified. This can be carried out by determining individual CpG methylation rates as can be done, for example, in a relatively imprecise manner, by sequencing or else, in a very precise manner, by a methylation-sensitive "primer extension reaction". It is also possible for the entire methylation status to be analyzed simultaneously, and for the patterns to be compared, for example, by clustering analyses which can be carried out, for example, by a computer.

Subsequently, it is possible to allocate the examined patients to a specific therapy group and to treat these patients selectively with an individualized therapy.

Example 2 can be carried out, for example, for the following diseases:
Adenosine deaminase deficiency, Viral infection, Retroviral infection, Sezary syndrome, Hematological disorders, Immunological disorders, Werner syndrome, Tuberculosis, Developmental disorders, Psoriasis, Rieger syndrome, Neurological disorders, Neurodegenerative disorders, Waardenburg syndrome, Niemann-Pick disease, Myelodysplastic syndrome, Myocardial infarction, Hypertension, Angiogenesis, Erythropoiesis, Congenital heart disease, HDR syndrome, Myelodysplastic syndrome, Arthiritis, Polyglutamine disorders, solid tumors and cancer

**Table 1**

| Listing of particularly preferred genes of the present invention associated with DNA transcription | |
|---|---|
| ***Gene*** | ***Database Entry No. Genbank at http:*//*www.ncbi.nlm.nih.gov)*** |
| ELF1 | M82882 |
| ETV3 | L16464 |
| ETV4 | D12765 |
| TAF2C2 | Y09321 |
| TCF9 | M29204 |
| ZNF121 | M99593 |
| ZNF131 | U09410 |
| ZNF139 | U09848 |
| ZNF 154 | U20648 |
| ZNF169 | U28322 |
| ZNF2 | X60152 |
| ZNF204 | AF033199 |
| ZNF206 | AA206569 |
| ZNF3 | X78926 |
| ZNF37A | X69115 |
| ZNF44 | X16281 |
| ZNF8 | M29581 |
| ZK1 | NM_005815 |
| ADA | NM_000022 |
| ATBF1 | NM 006885 |
| ATF3 | NM_001674 |
| ZNF255 | NM_005774 |
| CBFB | NM_001755 |
| CEBPD | NM 005195 |
| CTPS | NM_001905 |
| DCTD | NM_001921 |
| DR1 | NM 001938 |
| ELF3 | NM_00443 |
| ELK4 | NM_021795 |
| ETV5 | NM 004454 |
| KLF4 | NM_004235 |
| FOXO1A | NM_002015 |
| FLI1 | NM_002017 |
| HHEX | NM 001529 |
| HIVEP 1 | NM 002114 |
| HMG2 | NM_002129 |
| ID1 | NM_002165 |
| ID3 | NM 002167 |
| LAF4 | NM_002285 |
| ZNFN 1A1 | NM 006060 |
| LYL1 | NM_005583 |
| MAFG | NM 002359 |
| MAZ | NM 002383 |
| ODC 1 | NM 002539 |
| PBX3 | NM 006195 |
| POU2AF 1 | NM 006235 |
| POU2F2 | NM 002698 |
| POU3F1 | NM 002699 |
| PROP1 | NM 006261 |
| RARG | NM 000966 |
| RECQL | NM_002907 |
| RXRA | NM_002957 |
| SP100 | NM_003113 |
| ZFP93 | NM_004234 |
| MKRN3 | NM_005664 |
| ZNF133 | NM_003434 |
| ZNF157 | NM 003446 |
| ZNF173 | NM_003449 |
| ZNF189 | NM_003452 |
| ZNF207 | NM_003457 |
| ZNF262 | NM_005095 |
| ZNF264 | NM_003417 |
| ZNF74 | NM_003426 |
| ZNF91 | NM_003430 |
| POU3F2 | NM_005604 |
| ZNF84 | NM_003428 |
| ERV3 3 | |
| TCF8 | |

## Claims

1. A nucleic acid comprising a sequence at least 18 bases in length of a segment of the chemically pretreated DNA of genes associated with DNA transcription according to one of the sequences taken from the group of SEQ ID NO: 127 to SEQ ID NO: 128 and sequences complementary thereto.

2. An oligomer, in particular an oligonucleotide or peptide nucleic acid (PNA)-oligomer, said oligomer comprising in each case at least one base sequence having a length of at least 9 nucleotides which hybridizes to or is identical to SEQ ID NO: 127 to SEQ ID NO: 128.

3. The oligomer as recited in Claim 2,
wherein the base sequence includes at least one CpG dinucleotide.

4. A method for ascertaining genetic and/or epigenetic parameters for the diagnosis and/or therapy of existing diseases or the predisposition to specific diseases by analyzing cytosine methylations,
**characterized in that** the following steps are carried out:
a) in a genomic DNA sample, cytosine bases which are unmethylated at the 5-position are converted, by chemical treatment, to uracil or another base which is dissimilar to cytosine in terms of hybridization behavior;
b) fragments of the chemically pretreated genomic DNA are amplified using sets of primer oligonucleotides according to Claim 2 and a polymerase, the amplificates carrying a detectable label;
c) amplificates are hybridized to a set of oligonucleotides and/or PNA probes;
d) the hybridized amplificates are subsequently detected.

5. The method as recited in Claim 4,
**characterized in that** the chemical treatment is carried out by means of a solution of a bisulfite, hydrogen sulfite or disulfite.

6. The method as recited in one of the Claims 4 or 5,
**characterized in that** the genomic DNA is obtained from cells or cellular components which contain DNA, sources of DNA comprising, for example, cell lines, biopsies, blood, sputum, stool, urine, cerebral-spinal fluid, tissue embedded in paraffin such as tissue from eyes, intestine, kidney, brain, heart, prostate, lung, breast or liver, histologic object slides, and all possible combinations thereof.

7. A kit comprising a bisulfite (= disulfite, hydrogen sulfite) reagent as well as oligonucleotides and/or PNA-oligomers according to one of the Claims 2 or 3.

8. A nucleic acid amplificate, produced by
a) chemical conversion of cytosine bases which are unmethylated at the 5-position into uracil or another base which is dissimilar to cytosine in terms of hybridization behavior in a genomic DNA-sample by means of a solution of a bisulfite, hydrogen sulfite or disulfite and subsequent alkaline hydrolysis, and
b) PCR-amplification of fragments from said pretreated genomic DNA using sets of at least two primer oligonucleotides and a polymerase, wherein the primer oligonucleotides comprise a base sequence having a length of at least 18 nucleotides which is reverse complementary to or is identical to a chemically pretreated DNA of a gene associated with apoptosis according to one of the SEQ ID NO: 127 to SEQ ID NO: 128 and sequences complementary thereto, wherein the fragments have a length of 100 - 2000 basepairs.

9. The use of a nucleic acid according to Claim 1 or 2, of an oligonucleotide or PNA-oligomer according to one of the Claims 2 through 3, of a kit according to Claim 7, in the diagnosis or therapy of solid tumors and cancer.
